Europäisches Patentamt

European Patent Office

Office européen des brevets

(19)

(11) Veröffentlichungsnummer: **0 284 837 B1**

## (12) EUROPÄISCHE PATENTSCHRIFT

(45) Veröffentlichungstag der Patentschrift: **30.12.92**

(51) Int. Cl.5: **F25C 1/00**

(21) Anmeldenummer: **88103595.0**

(22) Anmeldetag: **08.03.88**

(54) Vorrichtung zum schnellen und gleichmässigen Einfrieren von zähfliessenden Flüssigkeiten.

(30) Priorität: **02.04.87 DE 3711169**

(43) Veröffentlichungstag der Anmeldung:
**05.10.88 Patentblatt 88/40**

(45) Bekanntmachung des Hinweises auf die
Patenterteilung:
**30.12.92 Patentblatt 92/53**

(84) Benannte Vertragsstaaten:
**AT BE CH ES FR GB GR IT LI LU NL SE**

(56) Entgegenhaltungen:
**EP-A- 0 064 311**
**FR-A- 2 299 625**
**US-A- 3 162 019**
**US-A- 3 777 988**
**US-A- 4 077 227**

(73) Patentinhaber: **MESSER GRIESHEIM GMBH**
**Hanauer Landstrasse 330**
**W-6000 Frankfurt/Main(DE)**

(72) Erfinder: **Buchmüller, Jürgen**
**Horstdyk 47**
**W-4150 Krefeld(DE)**
Erfinder: **Weyermanns, Günther**
**Hans-Sachs-Strasse 18**
**W-5142 Hückelhofen 6(DE)**

## Beschreibung

Die Erfindung betrifft eine Vorrichtung zum kontrollierten Einfrieren von zähfließenden Flüssigkeiten nach dem Oberbegriff des Anspruches 1.

Flüssigkeiten können konserviert werden, indem sie in Beuteln oder Flaschen aus plastischem Material eingefroren und gegebenenfalls vakuumgetrocknet werden. Wenn es sich um empfindliche Flüssigkeiten mit organischen Komponenten handelt, muß das Einfrieren möglichst schnell und gleichmäßig, also kontrolliert, erfolgen, um Kälteschäden zu vermeiden. Dies trifft insbesondere dann zu, wenn es sich bei den organischen Komponenten um lebende Zellen handelt, beispielsweise um Bakteriensuspensionen. Bei einem unkontrollierten Einfrieren können hierbei die Zellwand und das Zellgewebe durch eine starke Eiskristallbildung zerstört werden.

Eine solche Bakteriensuspension besteht z. B. aus 95 % Wasser und 5 % lebenden Bakterien. Wird unkontrolliert eingefroren, kann die Überlebensrate dieser Zellen auf ein unvertretbar geringes Maß absinken. Aber auch Flüssigkeiten mit anderen organischen Komponenten, beispielsweise Eiweißlösungen, Vitaminlösungen und Impfseren, können durch unkontrolliertes Einfrieren geschädigt werden. Eine bewährte Methode, derartige Flüssigkeiten zwecks Konservierung einzufrieren besteht darin, das Einfrieren mit Hilfe eines tiefsiedenden verflüssigten Gases, in der Regel Stickstoff, vorzunehmen. Mit flüssigem Stickstoff als Kältemittel kann die beispielsweise in Beuteln oder Ampullen befindliche Flüssigkeit sehr schnell auf die gewünschte Gefriertemperatur abgekühlt werden, so daß z. B. keine Zeit für eine große Eiskristallbildung bleibt. Es erfordert aber dennoch einige Zeit, eine derartige Flüssigkeitsprobe von außen nach innen einzufrieren, wodurch in geringem Umfang unvermiedbare Kälteschäden und Konzentration von Bestandteilen im Flüssigkeitskern auftreten.

Aus der GB-PS 1 376 972 ist eine Vorrichtung bekannt, die ein sehr schonendes Einfrieren einer solchen Flüssigkeit, nämlich Flüssigei, gestattet. Mit dieser Vorrichtung werden aus dem Flüssigei Tropfen geformt, in ein Bad aus einem tiefsiedenden verflüssigten Gas geleitet und aus diesem in Form von gefrorenen Pellets entnommen. Wegen des kleinen Volumens der Tropfen kann die Flüssigkeit überaus schnell auf die angestrebte Temperatur durchgefroren werden. Dies wird noch durch den direkten Wärmeaustausch zwischen Flüssigkeit und Kühlmittel unterstützt, da trennende Zwischenwände zwischen Kühlmittel und einzugefrierender Flüssigkeit entfallen. Zudem ergibt sich durch die Kugelform der Tropfen ein für das gleichmäßige Gefrieren optimales Verhältnis von Oberfläche zu Volumen der Flüssigkeit. Die Tropfen werden mit einer peristaltischen Pumpe erzeugt, indem mit der einzugefrierenden Flüssigkeit gefüllte Schläuche periodisch zusammengedrückt werden. Hierdurch werden Flüssigkeitstropfen aus den Schläuchen ausgestoßen und durch Düsen in das Kältebad geleitet.

Diese Art der Tropfenerzeugung ist aufwendig. Die peristaltische Pumpe ist eine verhältnismäßig komplizierte und störanfällige Maschine, die ständige Überwachung erfordert. Da sie in unmittelbarer Nähe des Kühlmittelbades angeordnet ist, besteht die Gefahr, daß die Düsen vereisen. Wenn hohe Anforderungen an die Einhaltung eines bestimmten Pelletdurchmessers gestellt werden, treten ebenfalls Schwierigkeiten auf, da sich mit der bekannten Vorrichtung nur Pellets mit einigermaßen gleichen Durchmessern herstellen lassen. Für das kontrollierte Einfrieren empfindlicher Flüssigkeiten sind aber Tropfen und Pellets einheitlicher Größe Voraussetzung, da sich nur bei solchen einheitlichen Größen identische und damit kontrollierte Einfrierbedingungen realisieren lassen. Die bekannte Vorrichtung bietet auch nur beschränkte Möglichkeiten, nämlich über den Durchsatz, die Tropfengröße zu variieren. Wenn verschiedenartige Flüssigkeiten von unterschiedlicher Zähigkeit zu Pellets gefroren werden sollen, ist aber häufig die Zuordnung einer bestimmten Pelletgröße zu einer bestimmten Flüssigkeit wünschenswert.

Eine weitere Einrichtung dieser Art zeigt die US-A-4 077 227. Hierbei werden die Tropfen einzeln

aus einer Hohlnadel gedrückt und fallen durch einen elektrisch geladenen Ring. Dadurch werden sie gleichartig elektrisch aufgeladen und stoßen sich ab, solange sie als Tropfen auf dem flüssigen Stickstoff schwimmen. Eine Agglomeration der Tropfen wird dadurch verhindert. Auch diese Art der Tropfenbildung ist aufwendig und hat zudem nur für solche Flüssigkeiten Sinn, die als Flüssigkeitstropfen auf der Stickstoffoberfläche schwimmen, bevor sie als gefrorene Pellets in den flüssigen Stickstoff absinken.

Der Erfindung liegt die Aufgabe zugrunde, eine Vorrichtung zum kontrollierten Einfrieren von zähfließenden Flüssigkeiten zu gefrorenen Pellets zu schaffen, die es gestattet, Pellets mit nahezu gleichen Durchmessern herzustellen, die Pelletgröße zu variieren und die einfach, robust und weitgehend wartungsfrei ist.

Ausgehend von dem im Oberbegriff des Anspruches 1 berücksichtigten Stand der Technik ist diese Aufgabe erfindungsgemäß gelöst mit den im kennzeichnenden Teil des Anspruchs 1 angegebenen Merkmalen.

Eine vorteilhafte Weiterbildung der Erfindung ist im Anspruch 2 angegeben.

Die Bedingung, daß die zu gefrierende Flüssigkeit zähfließend sein muß, bedeutet lediglich, daß aus der Flüssigkeit Tropfen definierter Größe hergestellt werden können. Es sind also Flüssigkeiten mit einem sehr großen Zähigkeitsbereich für die erfindungsgemäße Vorrichtung geeignet, lediglich sehr dünnflüssige Flüssigkeiten sind ungeeignet. Wichtig ist, daß die Tropfen beim Fallen in das Kühlmittelbad genügend Zeit haben, die Kugelform anzunehmen. Andererseits dürfen die Tropfen auch nicht mit zu hoher Geschwindigkeit in das Bad aus beispielsweise flüssigem Stickstoff eindringen, da dabei die Kugelform der Tropfen beeinträchtigt würde. Neben der Größe der Tropfen ist daher deren Fallweg in das Bad aus flüssigem Stickstoff, also die Tropfhöhe, entscheidend für ein optimales Gefrieren der Flüssigkeit. Die jeweils optimale Tropfhöhe kann durch einfache Versuche für jede Flüssigkeit leicht er mittelt werden.

Ein weiteres wichtiges Kriterium für einen optimalen kontrollierten Gefrierprozeß ist die Verweilzeit in dem Bad aus flüssigem Kuhlmittel. Die Verweilzeit kann auf einfache Weise mittels eines an sich bekannten das Bad durchlaufenden Förderbandes eingestellt werden, welches Mitnehmer zum Transportieren der zu Pellets gefrorenen Flüssigkeitstropfen besitzt.

Die Zeichnungen veranschaulichen ein Ausführungsbeispiel der Erfindung.

Es zeigen:

Fig.1     eine gesamte Anlage zum Einfrieren und anschließender Vakuumtrocknung,

Fig.2     die Gefrier- und Tropfeinrichtung von Fig.1 in vergrößerter Darstellung,

Fig.3     einen Ausschnitt aus Fig.2,

Fig.4     ein Detail der Tropfeinrichtung.

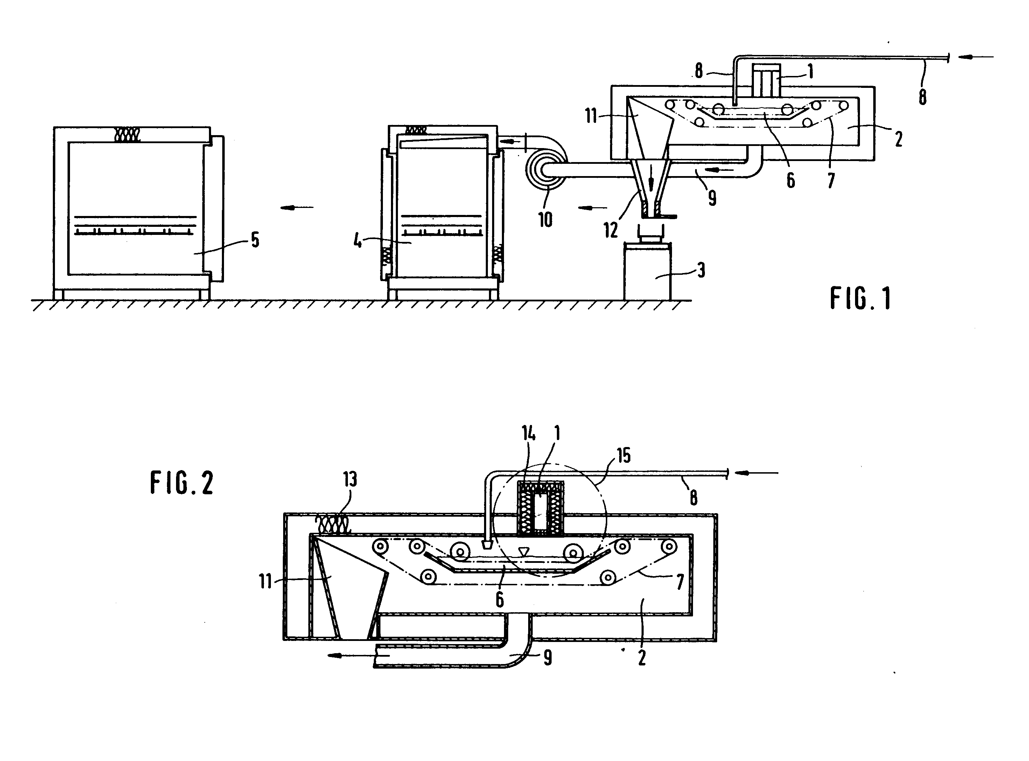

Die wesentlichen Teile der in Fig.1 dargestellten Anlage bestehen aus der Tropfeinrichtung 1, der Gefriereinrichtung 2, der Abfülleinrichtung 3, einem Gefrierschrank 4 und der Vakuumtrocknungseinrichtung 5. In der Tropfeinrichtung 1 befindet sich die zu gefrierende Flüssigkeit. Sie tropft in ein in der Gefriereinrichtung 2 befindliches Bad 6 aus flüssigem Stickstoff. Durch das Bad 6 läuft ein Förderband 7 mit einstellbarer Umlaufgeschwindigkeit. Durch Leitung 8 gelangt flüssiger Stickstoff in die Gefriereinrichtung 2, um den verdampften Stickstoff zu ersetzen. Der verdampfte Stickstoff wird aus der Gefriereinrichtung 2 durch das Rohr 9 mittels des Gebläses 10 abgezogen und dient dazu, den Gefrierschrank 4, der als Zwischenlager dient, auf einer geeigneten Temperatur von beispielsweise -60° C zu halten. Die Strömungsrichtungen und Bewegungsvorgänge sind durch nicht mit Bezugszeichen versehene Pfeile angegeben.

Die aus der erfindungsgemäßen Tropfeinrichtung 1 in das Bad 6 aus flüssigem Stickstoff tropfende Flüssigkeit gefriert dort zu Pellets und wird durch das Förderband 7 aus dem Bad 6 nach einer vorgegebenen Verweilzeit hinausbefördert. Die Pellets fallen durch die Trichter 11 und 12 in die Abfülleinrichtung 3, wo sie in Schalen abgefüllt werden. Die mit Pellets gefüllten Schalen gelangen zur Zwischenlagerung in den Gefrierschrank 4. Von dort gelangen sie in die Vakuumtrocknungseinrichtung 5, aus der das fertige Produkt entnommen werden kann.

Es ist selbstverständlich möglich, die aus dem Trichter 12 fallenden Pellets direkt in Beutel abzufüllen und in einem Gefrierlager bei beispielsweise -40° C aufzubewahren.

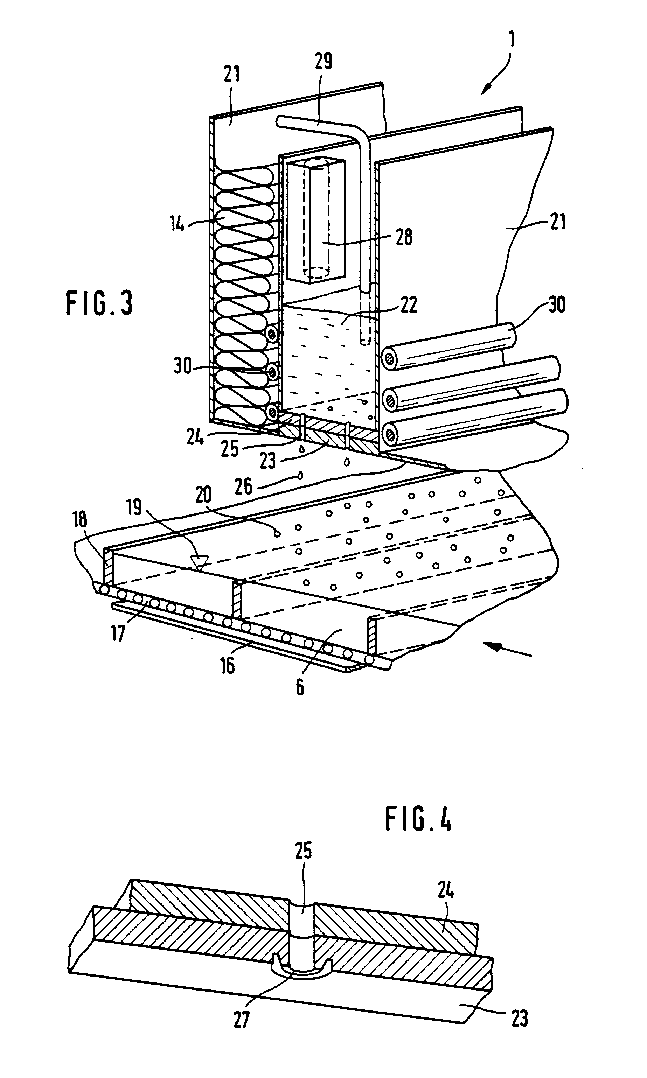

Fig. 2 zeigt die Gefriereinrichtung 2 in etwas vergrößerter Darstellung. Die gesamte Gefriereinrichtung 2 ist von einer Isolierung 13 umgeben, um die Kälteverluste gering zu halten. Auch die Tropfeinrichtung 1 besitzt eine Isolierung 14, um die einzugefrierende Flüssigkeit auf einer möglichst konstanten Temperatur halten zu können. Das durch den strichpunktierten Kreis 15 gekennzeichnete Detail ist in Fig. 3 in vergrößerter perspektivischer Darstellung dargestellt.

Fig. 3 zeigt einen Teil der Wanne 16, in welchem sich das Bad 6 aus flüssigem Stickstoff befindet. Durch das Bad 6 gleitet das Förderband 17, welches mit Mitnehmern 18 versehen ist. Die Oberfläche 19 des flüssigen Stickstoffes ist höher als die Mitnehmer 18. Zwischen den Mitnehmern 18 befinden sich die einzugefrierenden Pellets 20, die auf eine Kerntemperatur von -40° C eingefroren werden.

Oberhalb des Bades 6 aus flüssigem Stickstoff befindet sich die Tropfeinrichtung 1. Diese besteht im wesentlichen aus einem Behälter 21, in welchem sich die einzugefrierende Flüssigkeit 22 befindet. Der Boden des Behälters 21 besteht aus den Tropfscheiben 23 und 24. Die Tropfscheiben 23, 24 sind mit Bohrungen 25 versehen, die durch Verschieben einer Tropfscheibe, vorzugsweise der unteren Tropfscheibe 23, mehr oder weniger zur Deckung gebracht werden können. Hierdurch werden variable Drosselstellen gebildet, welche die Fließgeschwindigkeit der zu gefrierenden Flüssigkeit 22 so regeln, daß die Tropfenfolge bestimmt wird. Vorzugsweise sind die Tropfscheiben 23, 24 austauschbar, damit Tropfscheiben mit verschieden großen Bohrungen 25, je nach der Zähigkeit der einzugefrierenden Flüssigkeit 22 und gewünschter Tropfengröße verwendet werden können.

Fig. 4 zeigt in vergrößerter Ausführung in perspektivischer Darstellung Teile der Tropfscheiben 23 und 24 mit einer Bohrung 25. Die dem Bad zugewandte Kante der Bohrung 25 der unteren Tropfscheibe 23 ist als Abrißkante ausgebildet. Diese Abrißkante 27 ist eine Ringfläche, die durch Einfräsen einer Vertiefung um die Bohrung 25 gebildet wird. Je nach Zähigkeit der einzugefrierenden Flüssigkeit wird die Ringfläche größer oder kleiner gehalten.

In Fig. 3 sind noch weitere Einrichtungen dargestellt, die sicherstellen, daß die Tropfen 26 eine gleichbleibende Größe haben. So hält der Näherungsschalter 28 das Niveau der Flüssigkeit 22 auf konstanter Höhe und steuert die Flüssigkeitszufuhr durch die Leitung 29 entsprechend. Hierdurch wird erreicht, daß der Druck der Flüssigkeit 22 vor den Bohrungen 25 konstant ist. Statt des Näherungsschalters 28 können auch andere hierfür gebräuchliche Mittel verwendet werden. Der Behälter 21 ist ferner von einem Heizband 30 umgeben. Durch das Heizband 30 wird die Temperatur der Flüssigkeit 22 konstant gehalten.

Dies ist wichtig, weil die Zähigkeit der Flüssigkeit stark von der Temperatur abhängig ist und die Zähigkeit wiederum mit ausschlaggebend für die Zähigkeit der Tropfen 28 ist. Zugleich wird hiermit ein Zufrieren der Bohrungen 25 vermieden.

Mit der erfindungsgemäßen Einrichtung können demnach von einer vorgegebenen Flüssigkeit Tropfen konstanter und reproduzierbarer Größe gebildet werden. Für ein kontrolliertes Einfrieren ist es dann nur noch erforderlich, daß die Tropfen in Kugelform in das Stickstoffbad eindringen und daß die Verweilzeit im Stickstoffbad genau festgelegt werden kann. Die Verweilzeit wird ausschließlich durch die Umlaufgeschwindigkeit des Förderbandes 17 bestimmt. Die Mitnehmer 18 sorgen dafür, daß alle Tropfen 26 unverzüglich nach dem Eintauchen in den flüssigen Stickstoff durch das Bad 6 während einer vorbestimmten Zeit bewegt werden. Dabei werden sie zu Pellets mit der gewünschten Kerntemperatur gefroren. Um die angestrebte Kugelform zu erreichen, welche ein äußerst gleichmäßiges Einfrieren ermöglicht, muß die Fallhöhe der Tropfen 26 der jeweiligen Flüssigkeit entsprechend angepaßt werden. Sehr zähfließende Flüssigkeiten benötigen einen größeren Fallweg, da sie langsamer die Kugelform annehmen. Die günstigste Fallhöhe kann durch wenige Versuche schnell gefunden werden, da die Form der Pellets sofort Aufschluß darüber gibt, ob die Fallhöhe optimal ist.

Die erfindungsgemäße Vorrichtung ist für alle Flüssigkeiten geeignet, deren Zähigkeit eine kontrollierte und reproduzierbare Tropfenbildung zuläßt. So wurden erfolgreich Bakteriensuspensionen mit Feststoffanteilen zwischen 8 bis 16 Gew.% eingefroren. Diese Bakteriensuspensionen hatten Viskositäten zwischen 0,001 und 12,5 Ns/m$^2$ und Oberflächenspannungen zwischen 0,05 und 0,08 Ns/m. Je nach der Zähigkeit der einzugefrierenden Bakteriensuspensionen wurden Tropfscheiben mit unterschiedlich großen Bohrungen verwendet. Der kleinste Bohrungsdurchmesser betrug 0,7 mm, der größte Bohrungsdurchmesser 2 mm. Einen gewissen Einfluß auf die Größe der gebildeten Tropfen hat auch die Dicke der dem Stickstoffbad zugewandten Tropfscheibe. Wesentlich größeren Einfluß hat jedoch die jeweilige Tropfhöhe, also der Abstand der unteren Tropfscheibe vom Spiegel des Stickstoffbades. So wurden für den jeweiligen Einzelfall optimale Tropfhöhen zwischen 50 und 120 mm ermittelt. Der Durchmesser auf diese Weise gewonnenen kugelförmigen Pellets betrug zwischen 2 und 5 mm. Selbstverständlih ist dabei einer bestimmten Bakteriensuspension jeweils ein bestimmter Bohrungsdurchmesser, eine bestimmte Tropfhöhe und ein bestimmter Kugeldurchmesser zuzuordnen.

## Patentansprüche

1. Vorrichtung zum schnellen und gleichmäßigen Einfrieren von zähfließenden Flüssigkeiten (22) zu Pellets in einem Bad (6) aus einem tiefsiedenden verflüssigten Gas, mit einem oberhalb des Bades angeordneten Behälter (21) zur Aufnahme der einzufrierenden zähfließenden Flüssigkeit (22) und einer Einrichtung zur Versorgung des Bades (6) mit Tropfen (26) der Flüssigkeit,
dadurch gekennzeichnet,
daß der Boden des Behälters (21) aus zwei mit Bohrungen (25) versehenen gegeneinander verschiebbaren Tropfscheiben (23,24) besteht, Mittel zum Konstanthalten der Flüssigkeitshöhe im Behälter (21) vorhanden sind und ein den Behälter umgebendes Heizband (30) zur Konstanthaltung der Flüssigkeitstemperatur vorgesehen ist.

2. Vorrichtung nach Anspruch 1,
dadurch gekennzeichnet,
daß die der Badoberfläche zugewandten Kanten der Bohrungen der unteren Tropfscheibe als Abrißkanten (27) ausgebildet sind.

## Claims

1. Device for the rapid and uniform freezing of viscous liquids (22) into pellets in a bath (6) consisting of a low-boiling liquefied gas, with, arranged above the bath, a container (21) for receiving the viscous liquid (22) to be frozen and a device for supplying the bath (6) with drops (26) of the liquid, characterised in that the bottom of the container (21) consists of two drip plates (23, 24) which are provided with bores (25) and are displaceable in relation to one another, means are present for keeping constant the liquid level in the container (21) and a heating band (30) surrounding the container for keeping constant the liquid temperature is provided.

**2.** Device according to Claim 1, characterised in that the edges of the bores of the lower drip plate facing the bath surface are designed as breakaway edges (27).

**Revendications**

**1.** Dispositif de congélation rapide et régulière de liquides visqueux (22) pour former des boulettes, dans un bain (6) d'un gaz liquéfiable à basse température d'ébullition, avec un réservoir (21) pour recevoir le liquide (22) visqueux à congeler ainsi qu'une installation pour alimenter le bain (6) en gouttes (26) de liquide, dispositif caractérisé en ce que le fond du réservoir se compose de deux plaques d'égouttage (23, 24) munies de perçages (25) et coulissant l'une par rapport à l'autre, des moyens pour maintenir constante la hauteur du liquide dans le réservoir (21) ainsi qu'une bande chauffante (30) entourant le réservoir pour maintenir la température du liquide.

**2.** Dispositif selon la revendication 1, caractérisé en ce que le bord des perçages situés du côté de la surface du bain est réalisé en forme d'arête de décrochement (27) dans la plaque d'égouttage inférieure.

FIG. 1

FIG. 2

**FIG.3**

**FIG.4**